# EUROPEAN PATENT SPECIFICATION

(11) **EP 3 600 039 B1**
(45) Date of publication and mention of the grant of the patent: **24.05.2023**
(21) Application number: 18735091.3
(22) Date of filing: 08.06.2018
(51) Int. Cl.: A61B 5/1455, A61B 5/1495

(54) **SYSTEMS AND METHODS FOR DRIVING OPTICAL SENSORS**
SYSTEME UND VERFAHREN ZUM ANSTEUERN VON OPTISCHEN SENSOREN
SYSTÈMES ET PROCÉDÉS POUR ENTRAÎNER DES CAPTEURS OPTIQUES

(30) Priority: 09.06.2017 US 201762517564 P; 07.06.2018 US 201816003104
(43) Date of publication of application: 05.02.2020
(73) Proprietor: Covidien LP, Mansfield, MA 02048 (US)
(72) Inventor: BUXTON, William Kip, Arvado Colorado 80005 (US); EIKEL, Robert W., Lafayette Colorado 80026 (US); LI, Youzhi, Longmont Colorado 80503 (US); LISOGURSKI, Daniel, Boulder Colorado 80303 (US); MEEHAN, Christopher Joseph, Denver Colorado 80205 (US)
(74) Representative: Marks & Clerk LLP
(86) International application number: PCT/US2018/036619
(87) International publication number: WO 2018/227055

(56) References cited:
- US-A1- 2002 038 081
- US-A1- 2005 187 450
- US-A1- 2013 325 388
- US-B1- 6 356 774

## Description

### RELATED APPLICATIONS

This application claims the benefit of and priority to U.S. Provisional Application 62/517,564, filed on June 9, 2017, and entitled "Systems and Methods for Driving Optical Sensors". This application also claims the benefit of and priority to U.S. Application 16/003,104, filed on June 7, 2018, and entitled "Systems and Methods for Driving Optical Sensors".

### BACKGROUND

The present disclosure relates generally to medical devices, and more particularly, to medical devices that monitor physiological parameters of a patient, such as pulse oximeters.

In the field of medicine, doctors often desire to monitor certain physiological characteristics of their patients. Accordingly, a wide variety of devices have been developed for monitoring many such physiological characteristics. Such devices provide doctors and other healthcare personnel with the information they need to provide the best possible healthcare for their patients. As a result, such monitoring devices have become an indispensable part of modern medicine.

One technique for monitoring certain physiological characteristics of a patient uses attenuation of light to determine physiological characteristics of a patient. This is used in pulse oximetry, and the devices built based upon pulse oximetry techniques. Light attenuation is also used for regional or cerebral oximetry. Oximetry may be used to measure various blood characteristics, such as the oxygen saturation of hemoglobin in blood or tissue, the volume of individual blood pulsations supplying the tissue, and/or the rate of blood pulsations corresponding to each heartbeat of a patient. The signals can lead to further physiological measurements, such as respiration rate, glucose levels or blood pressure.

As technologies advance, and more measurements become available from the attenuated light signals, many caregivers find it convenient to have multiple physiological measurement parameters available in a single, multi-parameter monitoring device. However, such devices may have specific power requirements or limited available power.

Additionally, wireless, wearable monitors or sensors have been developed, to improve patient mobility and comfort, but these wireless devices may also have limited power available, such as through a rechargeable or disposable battery.

The remainder of this disclosure offers solutions and improvements for developing medical monitors, sensors, or modules that can operate effectively and reliably in low or reduced power environments, or in combinations of newer and older generation systems.

### OVERVIEW

Described herein are methods and apparatus for monitoring a patient. In one example, a sensor that is configured to detect physiological changes in a patient is driven at a first current level and a first sensor voltage is measured. Thereafter the current is varied to a second current level, and a second sensor voltage is measured. A next sensor voltage is projected based on the first sensor voltage and the second sensor voltage, and a third current level is identified that could produce the next sensor voltage. The third current level is compared to a threshold, and an alarm is produced in response thereto. The apparatus further includes elements in the sensor that facilitate the method.

Further described is a method of manufacturing a sensor. According to an example a communication interface is provided, through which a sensor can communicate with a monitor. A detector, capable of detecting light from a patient, is communicatively coupled to a memory, which is also communicatively coupled to the communication interface. Calibration data relating to one or more wavelengths of one or more light emitting elements is stored in the memory along with drive information relating to one or more input specifications for one or more of the light emitting elements. US 2013/325388 describes determining first and second values for a sensor feature parameter of a physiological sensor, comparing the first and second values, and making a decision on acceptance of the physiological sensor based on the comparison.

### SUMMARY OF THE INVENTION

The present invention provides a method of initializing a sensor for monitoring a patient as defined in claim 1.

The present invention further provides a patient monitoring system as defined in claim 10.

Preferred embodiments of the invention are defined in the dependent claims.

### BRIEF DESCRIPTION OF THE DRAWINGS

Advantages of the disclosed techniques may become apparent upon reading the following detailed description and upon reference to the drawings in which:
FIG. 1 illustrates a perspective view of a patient monitoring system including a patient monitor and a sensor, in accordance with an embodiment;
FIG. 2 illustrates a block diagram of the patient monitoring system of FIG. 1, including the patient monitor and the sensor of FIG. 1, in accordance with an embodiment;
FIG. 3 illustrates a chart of drive voltage available from the monitor of FIG. 2, in accordance with an embodiment;
FIG. 4 illustrates a schematic diagram of an equivalent circuit for an LED, in accordance with an embodiment;
FIG. 5 illustrates a graph of V/I curves for a number of different LEDs, in accordance with an embodiment;
FIG. 6 illustrates a graph showing the intersection between the V/I curves for a number of LEDs and the V/I curves of drive voltage available from the monitor, in accordance with an embodiment;
FIG. 7 illustrates a method for driving a sensor in accordance with an embodiment;
FIG. 8 illustrates a method for driving a sensor in accordance with an embodiment;
FIG. 9 illustrates a graph showing the intersection between the V/I curve for an LED with and without added buffer/headroom and the V/I curve of drive voltage available from a monitor, in accordance with an embodiment;
FIG. 10 illustrates a perspective view of a patient monitoring system including a patient monitor, a sensor and an adapter, in accordance with an embodiment;
FIG. 11 illustrates a method for driving a sensor in accordance with an embodiment;
FIG. 12 illustrates a method for driving a sensor in accordance with an embodiment; and
FIG. 13 illustrates a method of manufacturing a sensor in accordance with and embodiment.

### DETAILED DESCRIPTION OF THE SPECIFIC EMBODIMENTS

The present invention relates to medical sensors and monitors, and in particular to systems and methods for dynamically testing and confirming sensor operating range, which can be useful in low power or controlled power environments. Systems and methods are described for a safety check that measures available voltage or current in a medical monitoring environment, tests a sensor to evaluate its performance characteristics in that environment, and assesses that testing against minimum quality and safety standards. Sensors that do not meet the minimum quality standards are rejected as incompatible with the medical monitor, so that a medical caregiver can select a different, compatible sensor instead to ensure patient safety will not be compromised due to degraded performance.

This type of dynamic testing and sensor assessment can be particularly useful in low power applications, such as wearable or wireless monitors or sensors, where the available power is limited or reduced. For example, a wearable monitor may have a small, compact size in order to be wearable by a patient, and as a result may utilize a small battery that has a limited available voltage. At the same time, individual electrical components that are available for these systems are continually improving or advancing, which may result in changes to their power consumption profiles. For example, light emitting components such as LED's can be driven to higher brightness at lower currents than in the past. However, interactions between changing power consumption and constrained power availability can lead to incompatibility between certain components during active monitoring. In order to prevent system failures during critical medical procedures, systems and methods described herein evaluate system compatibility to make sure the medical monitoring system meets safety and quality standards and has a valid operating range.

One or more specific embodiments of the present techniques will be described below. In an effort to provide a concise description of these embodiments, not all features of an actual implementation are described in the specification. It should be appreciated that in the development of any such actual implementation, numerous implementation-specific decisions must be made, which may vary from one implementation to another. In an embodiment, a medical monitoring system includes a sensor that is actively powered during use.

As technology moves towards lower power patient monitors, the power available to drive sensors is reduced. In some cases, the voltage available at a sensor drive line may be insufficient to properly drive an active sensor to the power level needed during patient monitoring. Thus, a process is set forth herein, by which the patient monitor may test the system, including the sensor or sensors, to determine compatibility and operating parameters of the system components. As part of this process, the patient monitor may test the system at a variety of current levels, and determine the voltage/current (V/I) curve and/or relationship for one or more points in the patient monitoring system. Additionally, this measurement may be stored in an encoder within the sensor, which may facilitate testing for incompatible sensors, as described in more detail below.

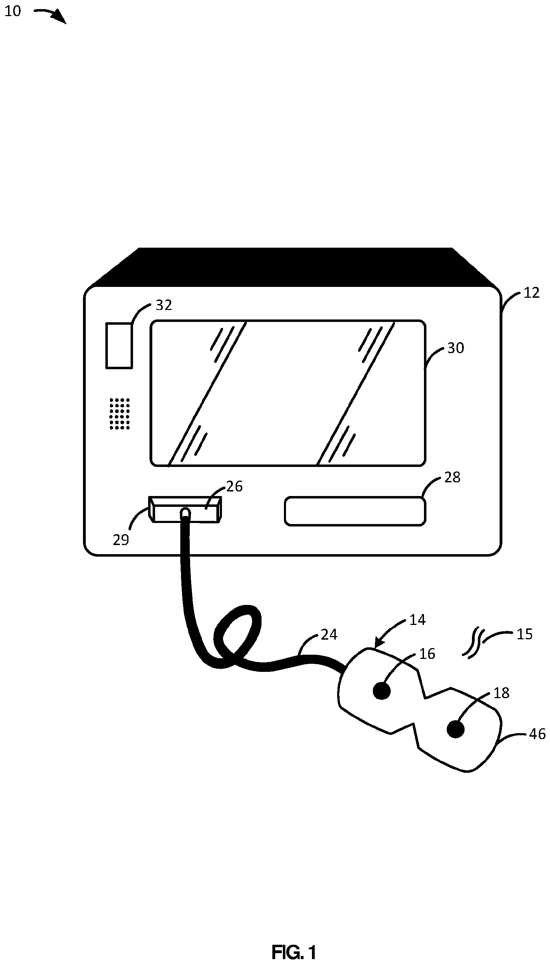

FIG. 1 illustrates an embodiment of a patient monitoring system 10 that may include a patient monitor 12 and a sensor 14, such as a pulse oximetry sensor, to monitor physiological parameters of a patient. By way of example, the sensor 14 may be a NELLCOR^{™}, or INVOS^{™} sensor available from Medtronic (Boulder, CO), or another type of oximetry sensor. Although the depicted embodiments relate to sensors for use on a patient's fingertip, toe, or earlobe, it should be understood that, in certain embodiments, the features of the sensor 14 as provided herein may be incorporated into sensors for use on other tissue locations, such as the forehead and/or temple, the heel, stomach, chest, back, or any other appropriate measurement site. In addition, although the embodiment of the patient monitoring system 10 illustrated in FIG. 1 relates to oximetry, the patient monitoring system 10 may be configured to obtain a variety of medical measurements with a different type of active medical sensor, such as an electroencephalography sensor, a temperature sensor, a blood pressure sensor, or any other actively powered sensor used to measure other physiological parameters (examples include tissue water fraction, respiration rate, or hematocrit).

In the embodiment of Figure 1, the sensor 14 is a pulse oximetry sensor that includes one or more emitters 16 and one or more detectors 18. For pulse oximetry applications, the emitter 16 transmits at least two wavelengths of light (e.g., red and/or infrared (IR)) into a tissue of the patient. For other applications, the emitter 16 may transmit 3, 4, or 5 or more wavelengths of light into the tissue of a patient. The detector 18 may be a photodetector selected to receive light in the range of wavelengths emitted from the emitter 16, after the light has passed through the tissue. Additionally, the emitter 16 and the detector 18 may operate in various modes (e.g., reflectance or transmission). In certain embodiments, the sensor 14 may include sensing components in addition to, or instead of, the emitter 16 and the detector 18. For example, in one embodiment, the sensor 14 may include one or more actively powered electrodes (e.g., four electrodes) to obtain an electroencephalography signal. The sensor 14 also includes a sensor body 46 to house or carry the components of the sensor 14. The sensor 14 may be reusable (such as a durable plastic clip sensor), disposable (such as a fabric adhesive sensor), or partially reusable and partially disposable.

In the embodiment shown, the sensor 14 is communicatively coupled to the patient monitor 12. In certain embodiments, the sensor 14 may include a wireless module configured to establish a wireless communication 15 with the patient monitor 12 using any suitable wireless standard. For example, the sensor 14 may include a transceiver 41 that enables wireless signals to be transmitted to and received from an external device (e.g., the patient monitor 12, a charging device, etc.). The transceiver may establish wireless communication 15 with a transceiver of the patient monitor 12 using any suitable protocol. For example, the transceiver may be configured to transmit signals using one or more of the ZigBee standard, 802.15.4x standards WirelessHART standard, Bluetooth standard, IEEE 802.11x standards, or MiWi standard. Additionally, the transceiver may transmit a raw digitized detector signal, a processed digitized detector signal, and/or a calculated physiological parameter, as well as any data that may be stored in the sensor, such as data relating to wavelengths of the emitters 16, or data relating to input specification for the emitters 16, as discussed below. Additionally, or alternatively, the emitters 16 and detectors 18 of the sensor 14 may be coupled to the patient monitor 12 via a cable 24 through a plug 26 (e.g., a connector having one or more conductors) coupled to a sensor port 29 of the monitor. In certain embodiments, the sensor 14 is configured to operate in both a wireless mode and a wired mode. Accordingly, in certain embodiments, the cable 24 is removably attached to the sensor 14 such that the sensor 14 can be detached from the cable to increase the patient's range of motion while wearing the sensor 14.

The patient monitor 12 is configured to calculate physiological parameters of the patient relating to the physiological signal received from the sensor 14. For example, the patient monitor 12 may include a processor configured to calculate the patient's arterial blood oxygen saturation, tissue oxygen saturation, pulse rate, respiration rate, blood pressure, blood pressure characteristic measure, autoregulation status, brain activity, and/or any other suitable physiological characteristics. Additionally, the patient monitor 12 may include a monitor display 30 configured to display information regarding the physiological parameters, information about the system (e.g., instructions for disinfecting and/or charging the sensor 14), and/or alarm indications. The patient monitor 12 may include various input components 32, such as knobs, switches, keys and keypads, buttons, etc., to provide for operation and configuration of the patient monitor 12. The patient monitor 12 may also display information related to alarms, monitor settings, and/or signal quality via one or more indicator lights and/or one or more speakers or audible indicators. The patient monitor 12 may also include an upgrade slot 28, in which additional modules can be inserted, so that the patient monitor 12 can measure and display additional physiological parameters.

Because the sensor 14 may be configured to operate in a wireless mode and, in certain embodiments, may not receive power from the patient monitor 12 while operating in the wireless mode, the sensor 14 may include a battery to provide power to the components of the sensor 14 (e.g., the emitter 16 and the detector 18). In certain embodiments, the battery may be a rechargeable battery such as, for example, a lithium ion, lithium polymer, nickel-metal hydride, or nickel-cadmium battery. However, any suitable power source may be utilized, such as, one or more capacitors and/or an energy harvesting power supply (e.g., a motion generated energy harvesting device, thermoelectric generated energy harvesting device, or similar devices).

As noted above, in an embodiment, the patient monitor 12 is a pulse oximetry monitor and the sensor 14 is a pulse oximetry sensor. The sensor 14 may be placed at a site on a patient with pulsatile arterial flow, typically a fingertip, toe, forehead or earlobe, or in the case of a neonate, across a foot. Additional suitable sensor locations include, without limitation, the neck to monitor carotid artery pulsatile flow, the wrist to monitor radial artery pulsatile flow, the inside of a patient's thigh to monitor femoral artery pulsatile flow, the ankle to monitor tibial artery pulsatile flow, and around or in front of the ear. The patient monitoring system 10 may include sensors 14 at multiple locations. The emitter 16 emits light which passes through the blood perfused tissue, and the detector 18 photoelectrically senses the amount of light reflected or transmitted by the tissue. The patient monitoring system 10 measures the intensity of light that is received at the detector 18 as a function of time.

A signal representing light intensity versus time or a mathematical manipulation of this signal (e.g., a scaled version thereof, a log taken thereof, a scaled version of a log taken thereof, etc.) may be referred to as the photoplethysmograph (PPG) signal. In addition, the term "PPG signal," as used herein, may also refer to an absorption signal (i.e., representing the amount of light absorbed by the tissue) or any suitable mathematical manipulation thereof. The amount of light detected or absorbed may then be used to calculate any of a number of physiological parameters, including oxygen saturation (the saturation of oxygen in pulsatile blood, SpO2), an amount of a blood constituent (e.g., oxyhemoglobin), as well as a physiological rate (e.g., pulse rate or respiration rate) and when each individual pulse or breath occurs. For SpO2, red and infrared (IR) wavelengths may be used because it has been observed that highly oxygenated blood will absorb relatively less Red light and more IR light than blood with a lower oxygen saturation. By comparing the intensities of two wavelengths at different points in the pulse cycle, it is possible to estimate the blood oxygen saturation of hemoglobin in arterial blood, such as from empirical data that may be indexed by values of a ratio, a lookup table, and/or from curve fitting and/or other interpolative techniques.

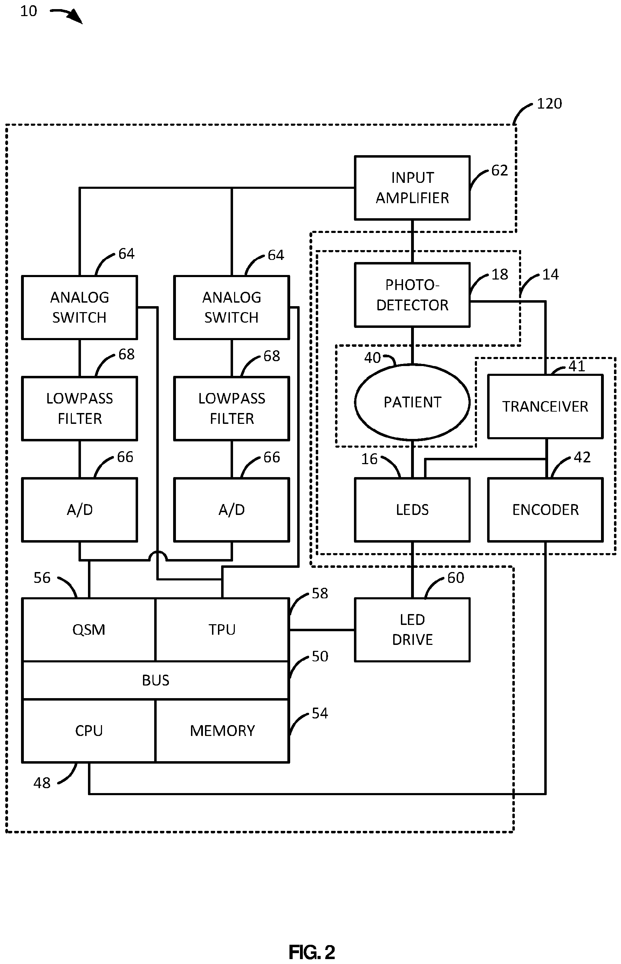

FIG. 2 is a block diagram of a patient monitoring system, such as patient monitoring system 10 of FIG. 1, which may be coupled to a patient 40 in accordance with an embodiment. Certain illustrative components of sensor 14 and patient monitor 12 are illustrated in FIG. 2.

Sensor 14 may include the emitter 16, the detector 18, and encoder 42. In the embodiment shown, emitter 16 is configured to emit at least two wavelengths of light (e.g., Red and IR) into the tissue of a patient 40. Hence, emitter 16 may include a Red light emitting light source such as Red light emitting diode (LED) and an IR light emitting light source such as IR LED for emitting light into the tissue of the patient 40 at the wavelengths used to calculate SpO2. In some embodiments, the Red wavelength may be between about 600 nm and about 700 nm, and the IR wavelength may be between about 800 nm and about 1000 nm.

It will be understood that, as used herein, the term "light" may refer to energy produced by radiation sources and may include one or more of radio, microwave, millimeter wave, infrared, visible, ultraviolet, gamma ray or X-ray electromagnetic radiation. As used herein, light may also include electromagnetic radiation having any wavelength within the radio, microwave, infrared, visible, ultraviolet, or X-ray spectra, and that any suitable wavelength of electromagnetic radiation may be appropriate for use with the present techniques. Detector 18 may be chosen to be specifically sensitive to the chosen targeted energy spectrum of the emitter 16.

In some embodiments, detector 18 may be configured to detect the intensity of light at the Red and IR wavelengths. In operation, light enters detector 18 after passing through the tissue of the patient 40. Detector 18 converts the intensity of the received light into an electrical signal. The light intensity is directly related to the absorbance and/or reflectance of light in the tissue of a patient 40. That is, when more light at a certain wavelength is absorbed or reflected, such as when a bolus of blood is passing through the tissue due to a heartbeat, less light of that wavelength is received from the tissue by the detector 18. After converting the received light to an electrical signal, the sensor 14 sends the signal to patient monitor 12, where physiological parameters such as SpO2, pulse rate, respiration rate, and other parameters are calculated based on the electrical signal.

In some embodiments, encoder 42 contains information about sensor 14, such as what type of sensor 14 it is (e.g., whether the sensor is intended for placement on a forehead, ear, nose, digit, etc.) and the calibration coefficients correlated with emitter 16. This information may be used by patient monitor 12 to select appropriate algorithms, lookup tables and/or calibration coefficients for calculating the patient's physiological parameters. For example, in an embodiment, the encoder 42 stores the calibration coefficients of the LED's 16 on the sensor, and the patient monitor 12 uses the calibration coefficient data to calibrate the conversion of the detected electrical signal to oxygen saturation based on the relative absorption of those wavelengths of light by the tissue 40.

Encoder 42 may contain information specific to patient 40, such as, for example, the patient's age, weight, and diagnosis. This information about a patient's characteristics may allow patient monitor 12 to determine, for example, patient-specific threshold ranges in which the patient's physiological parameter measurements should fall and to enable or disable additional physiological parameter algorithms. This information may also be used to select and provide coefficients for equations from which measurements may be determined based at least in part on the signal or signals received at sensor 14. For example, some pulse oximetry sensors rely on equations to relate an area under a portion of a PPG signal corresponding to a physiological pulse to determine blood pressure. These equations may contain coefficients that depend upon a patient's physiological characteristics as stored in encoder 42. Encoder 42 may, for instance, be a coded resistor that stores values corresponding to the type of sensor 14, the wavelengths of light emitted by emitter 16 on the sensor 14, and/or the patient's characteristics (such as adult, pediatric, neonate, weight range, or other characteristic). In some embodiments, encoder 42 may be a digital memory which stores this information. Specifically, the encoder 42 may include specific details about the LEDs such as minimum or maximum currents that can or should be supplied to the LEDs, or an indication of a voltage/current (V/I) curve relating to one or more LEDs, for example, as described in more detail below.

Signals from detector 18 and encoder 42 are transmitted to monitoring components 120, which may be housed in the patient monitor 12. In the embodiment shown, monitoring components 120 include a microprocessor 48 connected to an internal bus 50. Microprocessor 48 is adapted to execute specialized software, which may include an operating system and one or more applications, as part of performing the functions described herein. Also connected to bus 50 is a hardware memory 54, as well as user inputs, display, data output, and speaker, among others.

The monitor components 120 also include a time processing unit (TPU) 58 that provides timing control signals to light drive circuitry 60, which alternatively drives the Red LED and IR LED in emitter 16. TPU 58 may also control the gating-in of signals from detector 18 through amplifier 62 and switching circuits 64. These signals are sampled at the proper time, depending upon which light source is illuminated, producing two time-varying electrical signals representing the detected Red and IR light, respectively. Each received signal passes through amplifier 62, a low pass filter 68, and an analog-to-digital converter 66. The digital data may then be stored in a queued serial module (QSM) 56 (or buffer) for later downloading to memory 54. In some embodiments, there may be multiple separate parallel paths having components equivalent to amplifier 62, filter 68, and/or A/D converter 66 for multiple light wavelengths or spectra received. The amplifier 62 may amplify these parallel paths independently or together. Any suitable combination of components (e.g., microprocessor 48, RAM 54, analog to digital converter 66, any other suitable component shown or not shown in FIG. 2) coupled by bus 50 or otherwise coupled (e.g., via an external bus), may be used.

The monitor components 120 include electrical components that deliver power to the sensor 14 (to drive the LED's 16), and that receive, filter, digitize, and process the resulting electrical signals from the detector 18. The monitor components 120 include the microprocessor 48 which processes the received signals to determine the patient's physiological parameters, such as SpO2, pulse rate, and/or respiration rate by using various algorithms and/or look-up tables. The microprocessor 48 also receives information from the encoder 42, as described above, and uses this information in processing the signals. The signals from the encoder may include, for example, encoded information relating to patient characteristics or characteristics of the sensor 14. In some embodiments, the microprocessor 48 also receives information via user inputs, and such information may include monitor settings, alarm settings, and patient characteristics (such as age, weight, height, diagnosis, medications, treatments, and so forth).

The patient monitor 12 in Figure 1 is shown as a stand-alone box with its own sensor port 29 and display 30. In this embodiment, the monitor components 120 (shown in the dotted-line box in Figure 2) are housed within the monitor 12 to communicate with the sensor 14, and calculate and display physiologic parameters of the patient 40. Alternatively, the monitor components 120 can be provided on an independent circuit board that is itself integrated into a larger, multi-parameter patient monitor. The monitor components 120 on the circuit board may be one of many separate circuit boards that are integrated into the multi-parameter monitor to collect many different signals and data together into one monitor display. In this case, the monitoring components 120 can be provided in a miniaturized form factor, or carried by a circuit board, such that the monitoring components 120 can be connected with or integrated into the multi-parameter monitor. The multi-parameter monitor may be configured to calculate physiological parameters and to provide a central display for the visualization of information from the monitoring board (with the components 120) and/or from other medical monitoring devices or systems. The multi-parameter monitor includes a processor that is configured to execute code for calculating one or more physiological parameters and displaying the physiological parameters and/or other information about the patient monitoring system 10. In addition, the patient monitor 12, the multi-parameter monitor integrated with monitoring components 120, and/or the sensor 14 may be connected to a network to enable the sharing of information with servers or other workstations.

As the above descriptions show, the sensor 14 communicates with a larger system that includes other electrical components, processors, displays, and power sources. In normal operation, the power available to drive the sensor 14 may fluctuate as other devices are plugged into or removed from the system, such as other sensors or devices plugged into or removed from a multi-parameter monitor. The power available to drive the sensor can also change over time as batteries age or are depleted, or due to fluctuations in current from wall outlets. Moreover, components may be upgraded at different times, such that an updated circuit board (with monitoring components 120) may need to drive a range of different sensors (designed at different times, with various components), or a new sensor may need to be driven by a range of different monitors or circuit boards (also designed at different times, with various components). Solutions are described below that can facilitate updates to these components without scrapping large existing inventories, and that can enable cross-functionality between newer-generation and older-generation systems, while still maintaining a sufficient operating range to enable accurate patient monitoring. For example, solutions are described to enable a monitor to effectively operate different sensors that each have different power consumption and operating ranges, such as a first sensor connected by a cable and powered by the monitor as well as a second, newer wireless sensor connected by wireless communication and powered by its own battery.

Further, the sensor 14 itself varies its power requirements depending on the situation, in order to maintain a reliable signal for physiologic measurements. For example, the amount of attenuation of the optical signal caused by tissue can vary based on the physiology of the patient 40. Further, the optical signal attenuated by the tissue of patient 40 can be degraded by noise, among other sources. Darker skin pigmentation tends to make the light signal more attenuated, and low blood perfusion tends to make the light signal weaker and more difficult to detect clearly. One source of noise is ambient light that reaches the light detector 18. Another source of noise is electromagnetic (EM) interference from other electronic instruments. Movement of the patient also introduces noise and distorts the signal. For example, the contact between the detector 18 and the patient's skin, or the emitters 16 and the skin, can be temporarily disrupted when movement causes either to move away from the skin. Also, because blood is a fluid, it responds differently than the surrounding tissue to inertial effects, which may result in momentary changes in volume at the point to which the sensor 14 is attached.

Noise (e.g., from ambient light, EM interference, or patient movement) can degrade the electrical signal from the sensor and the resulting physiologic parameters relied upon by a care provider, without the care provider's awareness. This is especially true if the monitoring of the patient is remote, the noise is too small to be observed (such as small patient movements), or the care provider is watching the instrument or other parts of the patient, and not the sensor site.

As a result, it may be important to maintain a minimum signal to noise ratio (SNR) to obtain a reliable signal. It may be important that the light drive circuitry 60 drive the emitter 16 at a certain current level, considering the efficiency of the patient monitoring system 10, the available power, and the specifications of the sensor 14. Thus, when a sensor is in use on a patient, the LED drive 60 varies the current supplied to the LED's 16 in order to increase or decrease the brightness of the LED's, to maintain a reliable SNR. For example, the LED drive 60 may increase the current to increase the brightness when a patient 40 has low blood perfusion, when additional room lights are turned on, to overcome additional ambient light noise, or when the patient moves, to track the physiologic signal through motion noise. As another example, the monitor may decrease the current to decrease the brightness of the LED's when a patient has strong perfusion, creating a very strong signal, or when room lights are turned off, to avoid saturating the detector 18 with too bright a signal. This current control is dynamically adjusted in real-time to track a patient's physiologic signal.

The LED drive 60 balances several competing interests in deciding how much power to use to drive the emitter 16. For example, when the patient monitor 12 is incorporated into a multiparameter device, either as part of a system with multiple patient monitors, or in a single physical monitor, there may be several circuit boards representing several patient monitors within the single physical, multi-parameter monitor. This multi-parameter monitor may rely on battery power for temporary or ongoing power. There could be an incentive for a reduction in overall power consumed by the patient monitor 12. As a result, the voltage of the power available to the patient monitor 12 may be limited. In some cases, for example, the voltage available may be limited to 3.3V, or 3.0V or 2.7V. Limited available voltage for the patient monitor 12 can present design challenges for the patient monitor 12. Moreover, the patient monitor 12 may need to accommodate a wide range of available voltage, to facilitate its use in different multi-parameter monitors, hospital installations, or other systems, with a range of different sensors, and to maintain flexibility for future needs of systems that haven't yet been designed.

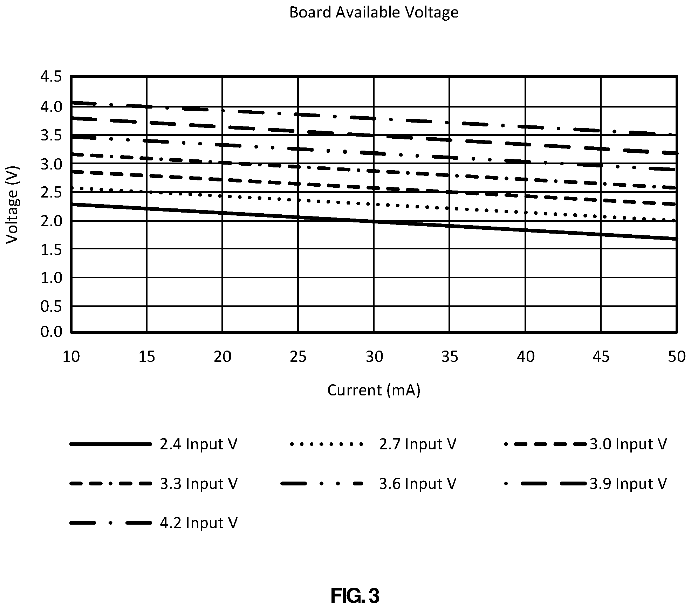

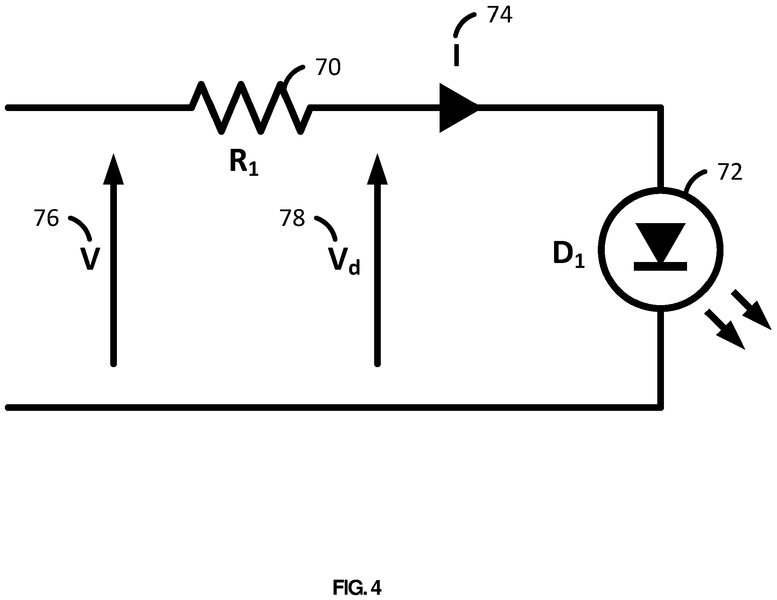

The voltage available from the monitor (from the emitter drive line, for example) to the sensor to drive the LED's varies depending on the available voltage to the monitor 12 (or monitoring components 120), as well as on the current being supplied to the sensor. This relationship is shown in Figure 3, which shows the same monitoring components 120 (such as in a patient monitor 12 or a multi-parameter monitor) operated at seven different supply line voltages (total voltage available to the board), which are labeled in the legend. Fixed voltages of 2.4V, 2.7V, 3.0V, 3.3V, 3.6V, 3.9V, and 4.2V are shown, though others can be used. Alternatively, a variable supply line voltage could be used. That is, in the top line trace, the circuit board 120 has a maximum of 4.2V available to it, and in the lowest line trace, the circuit board has a maximum of 2.4V. In an embodiment, the same monitoring components 120 are designed to operate in all of these different scenarios, to maintain flexibility for individual installations. However, as shown in Figure 3, even with a constant supply line voltage, the voltage that is available to the sensor to drive the LED's is not constant. Rather, the voltage available to the LED's drops as the current driving the emitter increases. In an embodiment, this is due to the internal resistance of the LED drive circuit 60 (see Figure 2), which consumes proportionately more voltage as current is increased. Thus, a board capped at 4.2V (top trace in Figure 4) can actually deliver only about 3.5V to the sensor LEDs when they are operated at a current of 50 mA.

The V/I curve in Figure 3 is based on a circuit model that models the resistance in the LED drive circuit 60. The voltage available from the monitor drive line to drive the emitter, or the V/I curve of the patient monitor drive circuit may be determined in accordance with a more complex circuit model as well. For instance, in an embodiment, the circuit model may include some capacitance or inductance between the supply voltage and the emitter drive, causing the V/I curve to be non-linear. There may be multiple drive circuits, causing the V/I curve to be piecewise linear, or piecewise non-linear. Additionally, the V/I curve may be determined through testing on the patient monitor, such that the data is determined empirically instead of through a model. Alternatively, the V/I curve could be determined using some combination of these methods. Further, the V/I curve could be determined at the time of designing the patient monitor, during testing, manufacture, or at any other time. Each individual board may have its V/I curve be determined independently, or the design of the board may use one generic V/I curve.

In some cases, this available voltage may be insufficient to drive all LEDs that may be available across a range of medical sensors. LEDs may react differently depending on the voltage and current applied, and at low available voltages, some LED's may not deliver adequate brightness to produce reliable physiologic signals, or may not have sufficient operating range to track a signal through noise, low perfusion, or other scenarios.

In considering the power tradeoffs discussed above, knowledge of the voltage available from the patient monitor drive circuit is only part of the information. The voltage required by the LED in order to effectively function is also part of the consideration. Turning to Fig. 4, most LEDs can be modeled by an equivalent circuit, as shown. While the equivalent circuit likely will not perfectly represent the LED, it does help to get a high-level understanding of how the LED will function when supplied with power. In an embodiment, an LED can be modeled as a Schottky diode 72 in series with a resistor 70. An LED could also be modeled using a different circuit model, as one of ordinary skill in the art would understand. The diode 72 is driven by a current 74, which is supplied at a given voltage 76. The LED has some internal resistance, represented by resistor 70, and voltage drops across a resistor 70 in relation to the current 74, in accordance with the equation V=I*R, where V is voltage, I is current, and R is resistance. Consequently, the voltage 76 is not the same as the voltage 78 seen across the diode. In many diodes, there is a minimum voltage 78 that must be present for the diode 72 to function. In the case of many LEDs, this can be in the range of 1.5 to 2.25 volts. In other cases, this minimum voltage 78 that is needed across the diode may be higher or lower.

While the voltage 78 seen across the diode will remain relatively constant while the diode is actively emitting light, the voltage 76 required across the LED will vary with the amount of current 74 supplied to the LED, in proportion to the resistance R1 of the LED. In some embodiments of the patient monitoring system 10, the current 74 supplied to the LED will vary over time, adjusted by the LED drive 60, as described above, in order to allow the patient monitoring system 10 to efficiently operate. The LED may have a minimum voltage below which the diode 72 may not function at all, or may produce inconsistent light. In some embodiments, this may lead to a situation in which the entire patient monitoring system 10 does not operate accurately. Thus, there may be value in determining the voltage available to drive the LEDs, as well as the minimum voltage 76 across the LED so that the diode will properly function.

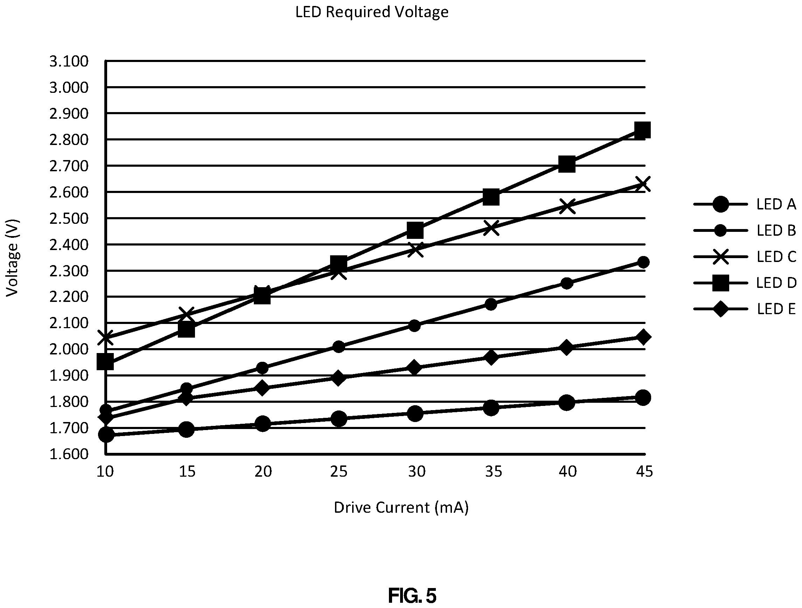

In Fig. 5 is shown the V/I curves for a number of LEDs. As can be seen, individual LEDs can vary significantly depending on their internal resistance (modeled by the resistor 70 in Figure 3). The LEDs represented in Fig. 5 include a variety of LED types, including LEDs from different manufacturers and LEDs of varying brightness. For example, LEDs D and E are the same model LED with slight variations in manufacturing. LED A is a legacy standard brightness LED, LED B is a legacy High brightness LED, and LED C is a new high efficiency, high brightness LED. The V/I curves for the LEDs can be determined in many ways. In an embodiment, each individual LED can be measured at a number of drive voltages to determine its V/I curve. As an option, the measurements could be made by measuring the voltage drop across the leads of the LED while being driven at a constant current. These measurements could occur at 2, 3, 4, 5 or more different drive currents. The manufacturer specification for the LED may also be used as a starting point, after which individual testing is used to individualize for the particular LED. As can be seen in Fig. 5, LED D, for example, requires an input voltage of over 2.8V at a 45mA drive current. As discussed above, at 45mA, this voltage may not be available from the emitter drive line of some patient monitors.

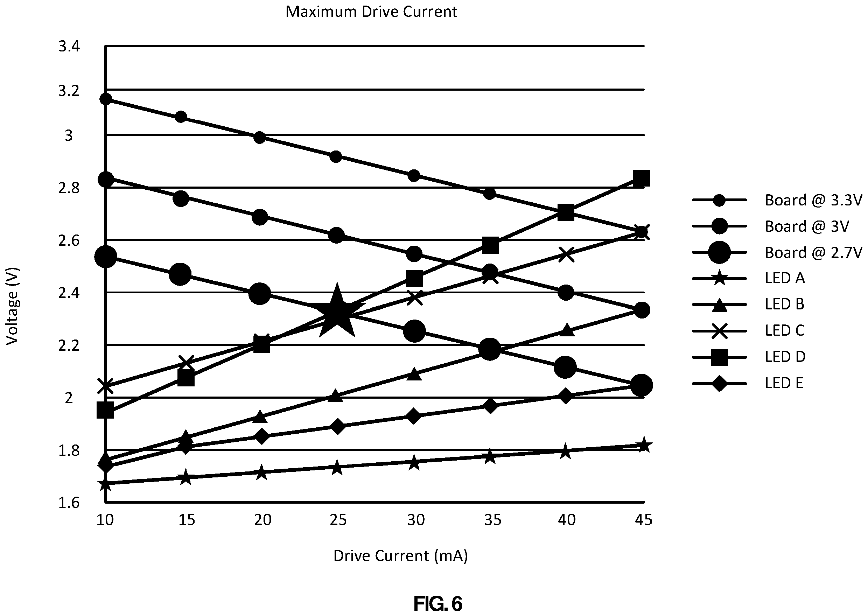

In Fig. 6 some of the patient monitor drive circuit V/I curves from Fig. 4 are shown together with the V/I curves for the LEDs from Fig. 5. It is apparent that, at higher drive currents, some of the LEDs in this example are incompatible with the patient monitors 12 modelled in this example. For example, at drive currents above 25mA, the input voltage requirements for both LEDs C and D are higher than the available drive voltage from the board at supply line voltage 2.7V. Similarly, at drive currents above 36mA, the input voltage requirements for LEDs B, C and D are higher than the voltage available from the patient monitor 12 with a supply line voltage of 2.7V. Looking at Figure 6 from the perspective of one of the circuit boards, the circuit board with an input voltage of 2.7V can drive a sensor all the way from 10 to 45 mA only if that sensor has LED A or LED E. The other sensors with the other LED's all reach a current level where they require more voltage than this circuit board can deliver.

In operation, if this circuit board is connected to a sensor with LED B, it may operate sufficiently at first, such as by operating the LED's below 35 mA. But if the monitoring conditions change and require a higher current to the LED's - such as if the patient's pulsatile signal becomes weak, or noise interferes, and the LED drive circuit 60 commands an increased current in order to maintain a suitable SNR in the physiologic signal - then the monitor may trigger a sensor error, if the commanded current is above what the monitor can deliver. This could result in the monitor discontinuing monitoring until a new sensor is connected. The loss of monitoring functionality could interfere with the patient's medical care.

According to an embodiment, a method is provided to check sensors for compatibility, in order to confirm that the sensor can be driven within a sufficiently wide operating range (for example, up to an adequate current level or SNR) in a given clinical environment, prior to using the sensor on a patient. This method reduces constraints on sensor and monitor development, enabling the use of newer, more advanced electrical components in both the sensor as well as monitors, by providing an additional safety check to verify that an individual sensor can be adequately operated in an individual monitor installation. Embodiments of this safety check are described below.

In an embodiment, the patient monitor (such as the stand-alone monitor 12 or monitoring components 120) conducts an initial test each time a sensor is connected to the monitor, to determine whether the monitor can supply enough voltage to drive the sensor to a minimum current level - which may be referred to herein as a check level or check value. This current check value is sensor-specific, based on the electrical properties of the particular emitters carried by the sensor. In an embodiment, this check value is programmed into each sensor and stored in the sensor's encoder 42. This check value defines a minimum operating range (between the check level and the absolute minimum current that can be used to cause the LED to activate) that is needed to be able to drive the sensor to various brightness levels to track the physiologic signal through noise, patient movement, and other situations.

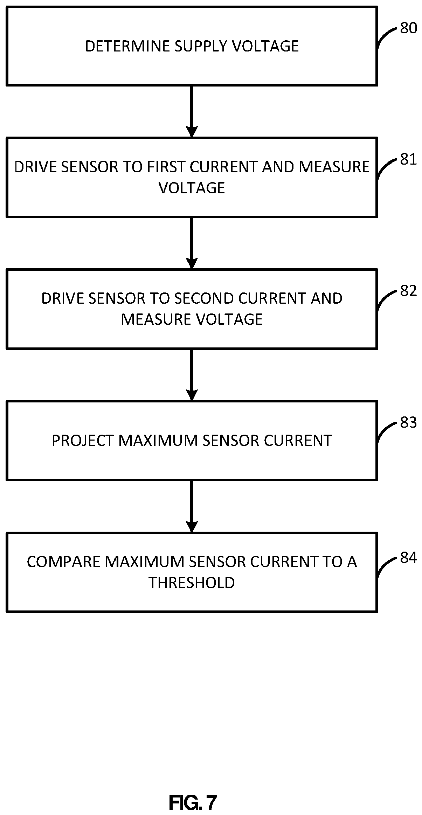

An example of this safety check is shown in Figure 7. Each time a sensor is connected to a monitor, the monitor treats the sensor as a new sensor and runs the safety check. In step 80, the safety check includes determining a supply voltage available from the emitter drive line. This supply voltage can vary with current, so the supply voltage will also be projected with current (such as in Fig. 4). The safety check further includes driving the sensor to a first current and measuring the forward voltage (V, item 76 in Figure 3) across the LED as shown in step 81. This voltage value is stored as the first voltage value. The safety check then includes step 82, driving the sensor to a second, different current and measuring a second, different forward voltage across the LED. The two measured current/voltage pairs are then used to determine the LED's V/I (voltage/current) curve (such as those shown in Figure 5). Next, the safety check includes step 83, projecting this LED V/I curve up to the point where it intersects the board's limited input voltage (as shown in Figure 6). For example, in Figure 6, the V/I curve of LED B intersects the 2.7V board's available voltage at about 35 mA and 2.2V. The current at this intersection is the maximum current that the board can deliver to the LED. This maximum current is compared against the minimum check value programmed into the sensor in step 84. If the projected maximum current is below the check value, then the sensor is rejected as defective. The check value programmed into the sensor can be considered a "minimum maximum" value (i.e. a minimum current that the board must be able to deliver in order for the sensor to have a sufficiently wide operating range and meet quality standards). While these steps are presented in a particular order in this example, in other implementations, these steps may be completed in a different order.

As an example, consider a sensor with LED D in Figure 6, connected to a board with 2.7V input voltage. The sensor's V/I curve is generated (such as by measuring its voltage at both 15 and 20 mA and fitting a line to those points), and this curve is plotted in Figure 6. Then, the intersection of the generated sensor V/I curve and the board's input voltage is found. For LED D and a 2.7V board, the intersection is indicated by the star in Figure 6. This intersection of the increasing sensor V/I curve with the decreasing board voltage shows the projected maximum current available for the LED (i.e. the maximum current that the board can deliver to the LED). For LED D and the 2.7V board, the projected maximum current is 25 mA. However, in an embodiment, the sensor with LED D is programmed with a check value of 30 mA, to make sure enough current is available to drive the LED's brightly enough to provide a quality PPG signal that enables accurate physiological measurements. As a result, this particular sensor is not compatible with the 2.7V board. Because the projected maximum current (25 mA) is below the minimum acceptable maximum current (check value - 30 mA), the sensor is rejected and the monitor issues an error message (or takes other steps as described below). A medical caregiver can then select a different sensor that is compatible with the monitor.

The minimum-maximum (check) value that is programmed into each sensor is determined based on the performance characteristics of that individual sensor. For example, a sensor with a new, brighter LED may be able to provide good performance at lower current values, and thus may be safe to operate in situations where the maximum available current is relatively low. Thus, a new sensor with a bright LED may have a lower value stored as its check value, compared to older sensors with less efficient LED's. The check value that is assigned to each sensor as the lowest maximum current for that sensor is based on quality and performance standards. These in turn can come from extensive testing of sensor performance, such as testing individual sensor components to assess the PPG signals that the sensor can produce at various current and voltage levels. A current value is selected that enables the sensor to deliver a PPG signal with a sufficient SNR, producing a discernible signal that can reliably be used for accurate physiological measurements, even in challenging patient conditions such as weak pulses, dark pigmentation, motion noise, and other interference. To provide reliable monitoring of the patient's vital signs even in these challenging conditions, the system needs to be able to drive the sensor up to that check value, providing enough current to the LEDs to increase their brightness and produce a sufficiently strong PPG signal. If the system can't reach the check value, then the sensor may not produce a quality PPG in challenging conditions, and the system rejects the sensor as faulty.

The check value can vary between sensor types, for example when different sensor types use different LED's with different brightness or power requirements. These different sensor types will have different V/I curves and different projected maximum currents. Moreover, even individual LED's that are the same type, with the same LED's and other electrical components, can vary based on minor differences in manufacturing or operating conditions, such as temperature. As a result, a sensor that fails the safety check may simply be an outlier from manufacturing, while other individual sensors of the same type will pass the safety check.

The patient monitor 12 or patient monitoring components 120 may be designed in such a way that it can determine what supply line voltage is supplied to it. This could be determined by a hardware setting when the patient monitor 12 is incorporated into the system, or by an algorithm run be the patient monitor 12 after it has been incorporated, for example. Optionally, both of these methods may be used, to corroborate each other.

Alternatively, the board V/I curve available at the emitter drive may be determined directly, particularly in the case of a variable supply line voltage. This may be accomplished by driving one or more of the LEDs at one or more set currents. If for example, the emitter drive line is driven at a target of 20mA, and then a target of 25mA, the board V/I curve for the emitter drive line could be determined. By way of example, the emitter drive line, which may be driven through a digital to analog convertor (DAC), for example, could be driven to a target of 20 mA. While the emitter drive is being powered by the patient monitor, the patient monitor 12 or a separate processor or piece of hardware could measure the actual current driven through emitter drive, as this may vary somewhat from the target of 20 mA. In order to increase accuracy, the actual current can be measured. In order to measure the voltage as well, the emitter drive could then be driven again at the same target current, and the voltage out of the emitter drive could be measured. This same procedure could then be carried out at a target of 25mA for example. As could be understood, the order of these measurements could be reversed. The data collected could then be used together with the supply line voltage to determine the maximum current that can be supplied by the patient monitor 12 to safely and effectively drive the LEDs. This process could be repeated for other currents levels as well.

In the safety check described above, the LED's V/I curve is generated dynamically by the patient monitor when the sensor is connected to the monitor. This enables the monitor to verify the sensor's performance (including projecting the maximum current value) in the operating environment of the monitor. This check allows determination of a safe and effective operation zone for the particular sensor 14 with the particular patient monitor 12. Since the amount of current that the patient monitor can supply can vary from sensor to sensor based on both the LED's V/I curve and the board V/I curve for the patient monitor emitter drive line, this check allows the patient monitor to determine the operating parameters available with a given combination of patient monitor 12 and sensor 14. Since the effectiveness of the LEDs can be compromised with current that is too low, this safety check also allows verification that the particular combination of patient monitor 12 and sensor 14 provides a window of operation (available variation of drive current) that is sufficiently broad to allow for effective patient monitoring.

In another embodiment, the sensor's V/I curve can be generated once at design or manufacture of the sensor if desired, and the measured V/I curve itself can be programmed into the sensor, such as stored on the encoder 42. The V/I curve can be stored as two measured voltage-current pairs, or a table of values, or an equation (such as a linear relationship based on two measured points). When the sensor is connected to a patient monitor, the monitor then projects along that V/I curve to find the intersection with the monitor's input voltage, as shown in Figure 6. As before, the monitor can then determine the maximum current available to the sensor, and check this against the check value programmed into the sensor.

In some cases, after the projected maximum drive current is determined, then the patient monitor 12 may physically attempt to drive the LED at that current. This step is taken because the projected maximum current is an estimate, and the actual performance of the system at that current may vary from the line-fit equation used for the sensor's V/I curve. If the emitter drive circuit is unable to drive the LED to the maximum drive current, then the estimate for the projected maximum current is reduced. The reduced value is then compared against the minimum value stored on the sensor, and if the reduced value is below the minimum, the sensor is rejected.

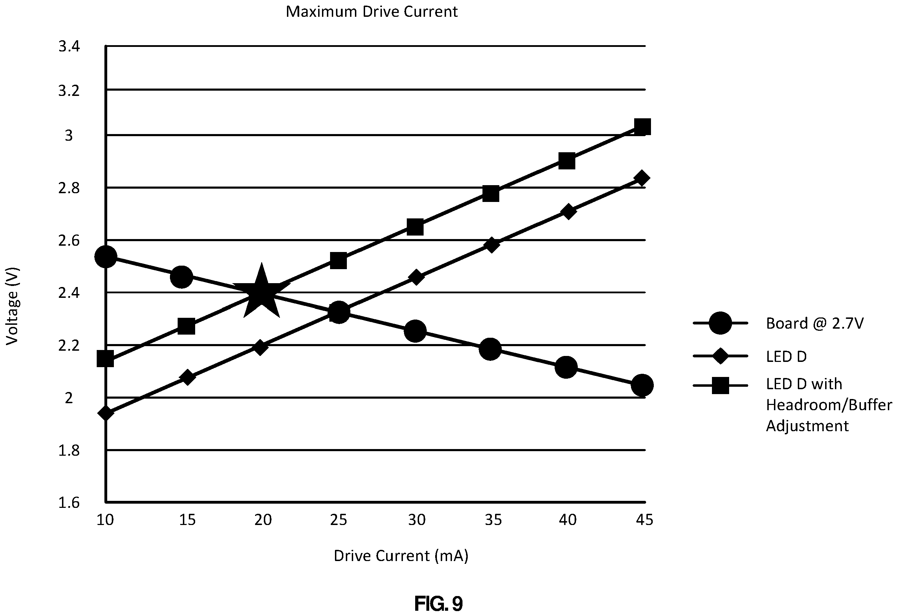

In accordance with an embodiment, a buffer or headroom allowance is also employed, essentially reducing the available operating range . The headroom is a buffer value that is added to the minimum required operating range. This accounts for operational space in which the LED may function, but do so sporadically or inconsistently. This headroom allowance could be a fixed buffer amount, such as 0.4V. For example, referring to Figure 9, the intersection of LED D with the 2.7V board has been replicated from Fig. 6. Additionally, a V/I curve for LED D is shown with a headroom or buffer adjustment. As seen in Figure 9, the headroom or buffer adjustment increases the entire V/I curve for LED D. For example, In Figure 9, a buffer value of 0.2V is added to the entire V/I curve for LED D to create the V/I curve for LED D with buffer/headroom adjustment. The V/I curve for LED D with the buffer adjustment intersects with the V/I curve for the 2.7V board at 20mA. Thus, the projected maximum current for LED D with a buffer/headroom adjustment when connected to this board is 20 mA, and this value is compared against the stored check value.

It should be apparent that this buffer or headroom adjustment can be alternatively be made to reduce the available operating range in other ways. For example, by reducing the voltage in the board available V/I curve, or by reducing the projected maximum current. In an embodiment, the headroom allowance may be adjustable. By way of example, the headroom allowance may be smaller for a supply line voltage of 2.7V, and higher for a supply line voltage of 3.3V. This variable headroom could vary between 0.3V and 0.4V for example.

Following calculation of a new maximum drive current which may include a headroom requirement, the LED drive may try again to drive the LED at this maximum drive current. As above, if the test is unsuccessful, then the maximum drive current may need to be reduced, and tried again. This can be repeated until the LED is successfully driven at its maximum current, with a resulting voltage that is below the headroom requirement.

In some cases, as described above, there may be a minimum allowable drive current bandwidth. An LED that is driven at very low currents may have inconsistent results, so the very low current end of the V/I curve could be unavailable for operation. Similarly, as discussed above, the upper current end of the V/I curve may not be available due to limited available emitter drive voltage (in comparison with LED V/I requirements) This may leave an available operational current band that is insufficient to effectively operate the patient monitor 12. If an LED could only be driven between 4mA and 12mA, for example, due to inconsistent results below 4mA, and insufficient emitter drive line voltage above 12mA, the 8mA current band available for operation of the patient monitor 12 may be too narrow for effective operation. Thus, in an effort to ensure consistency of the patient monitoring system, in an embodiment, the system requires a minimum current bandwidth, such as a 20 mA range, between the minimum and maximum currents. For example, where the minimum current that operates the LED is 4 mA, the system may require that the maximum be no less than 24 mA (4 mA plus the 20 mA bandwidth). This could ensure more flexibility for the patient monitor to adjust the drive current for the LED, producing safer and more effective monitoring results. In the embodiment discussed above, the sensor would be rejected as incompatible, since the emitter drive current can only be driven up to 12mA, instead of 24 mA (or other determined value).

In above examples, the monitor rejects an incompatible sensor. This may be done in a variety of ways, such as sounding an alarm, producing an error message, or disabling some functionality of the sensor. Alarms may be visual, audible, or tangible alarms, such as a vibration. Additionally, the patient monitor 12 may provide a simple notification to a patient or user through these alarms, or it may turn off some or all of its functionality in the case of an alarm. This alarming may be triggered by a simple threshold alarm, such as the maximum allowable drive current that a patient monitor 12 can supply to an LED is below a threshold or 25mA, for example. In this case, the patient monitor 12 could warn a patient that a sensor 14 may not function properly, or the patient monitor 12 may indicate that a sensor 14 is not functioning correctly, and cease functioning, for example. The alarming could also be based on a more complex threshold analysis involving multiple thresholds as well. For example, a sensor that fails the safety check may still be used to measure a patient's heart rate, but not SpO2. Alternatively, there could be a second threshold for a second measured parameter, such as a patient's heart rate. For example, the patient monitor 12 may require that the "minimum maximum" drive current be at least 25mA to measure Sp02 (or require a first, relatively larger current bandwidth for SpO2, such as a 20mA bandwidth), but the "minimum maximum" drive current only needs to be greater than 15mA to measure a patient's heart rate (or require a second, relatively smaller current bandwidth for heart rate, such as a 10 mA bandwidth). In this case, a sensor that has a "minimum maximum" drive current of 22mA in conjunction with a certain patient monitor 12 (for example, an 18mA range, from 4 to 22 mA) would be enabled to measure a patient's heart rate, but not SpO2. A sensor that has a "minimum maximum" drive current of 12mA (for example, an 8 mA range, from 4 to 12 mA) would not be enabled to measure either SpO2 or heart rate on the patient. This can allow a patient monitor to customize operation depending on the signal range necessary for a given measurement. According to an embodiment, when a sensor does not pass the safety check, the monitor may drive only the infrared LED emitter, and display only heart rate and pulse waveform on the monitor display. Any variety of parameters to measure and/or display may be selected for limited operation of sensor/monitor combination that falls below a safety check threshold.

Fig. 7 shows a method of operation according to an embodiment. In step 80 the patient monitor will determine the supply voltage. This is also a V/I curve reflecting the available voltage from the emitter drive line of the patient monitor 12. This determination could occur mathematically, using a fixed supply line voltage and a circuit model of the patient monitor to determine a V/I curve available from the patient monitor supply line. Alternatively, available voltage could be measured at two points and combined to determine a V/I curve. While this step is shown as the first step, the steps could occur in an alternative order in another implementation. Additionally, this step could happen before or after a sensor 14 is connected to a patient monitor 12.

When a sensor 14 is connected to a patient monitor 12, the patient monitor will drive the sensor at a first emitter drive current and measure the voltage at the emitter drive line. This step 81 may include a measurement of the emitter drive current as well as the voltage in order to increase accuracy. This could require two separate measurements - one measurement of current and one measurement of voltage. The patient monitor 12 will then drive the sensor 14 at a second emitter drive current and measure the voltage at the emitter drive line. As in step 81, step 82 could include measurement of current as well as voltage. The two measured V/I points are then used to calculate a V/I curve for the LED.

The patient monitor will then utilize both the V/I curve representing the LED requirements and the V/I curve representing the voltage available from the emitter drive line to find an intersection point in step 83. This point of intersection is the maximum drive current for this combination of sensor 14 and patient monitor 12. The patient monitor will compare this maximum drive current to a threshold to determine whether the operating range for the particular patient monitor 12 together with the sensor 14 is sufficiently broad. If the maximum drive current falls below a "minimum maximum" drive current threshold, then the patient monitor 12 will reject the sensor 14 in step 84.

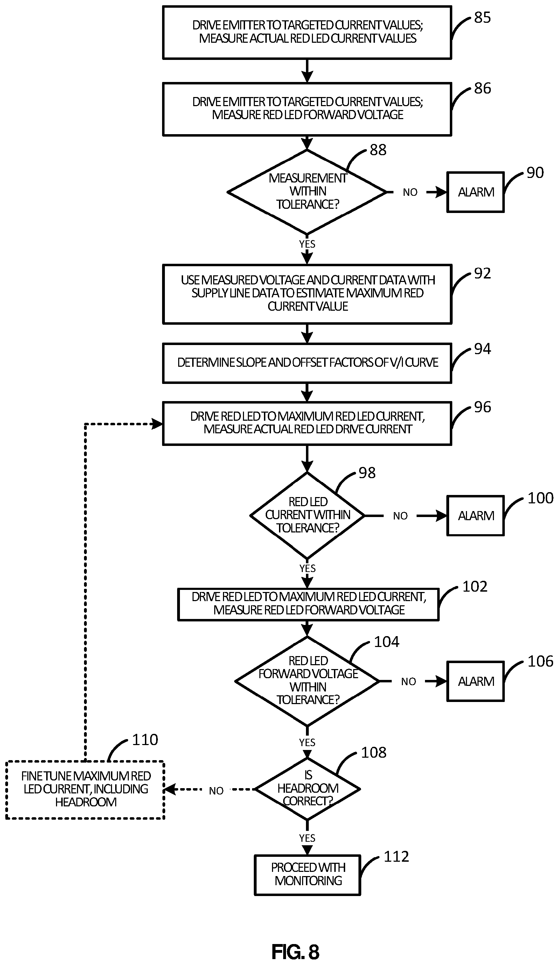

Fig. 8 shows a method of operation according to an embodiment. After connecting a sensor 14 to a patient monitor, the patient monitor 12 may drive the red emitter 16 to a first current level. In pulse oximetry, it is common for the patient monitor 12 to require more current variability on the red LED than on the IR LED, so in an embodiment, only the Red LED is analyzed for the safety check. The patient monitor 12 will attempt to drive the red emitter 16 at the first current level, but due to variations in manufacturing, environment, and implementation, the actual current level may vary from that first current level. So, in step 85, the patient monitor 12 attempts to drive the red emitter 16 at the first current level, and while driving the emitter, the actual current level is measured. This measurement may happen one time with one current level, or more than one time with multiple current levels.

Step 86 proceeds similarly to step 85, but measures the voltage across the red LED while the current is driven to a target level. This sensor voltage could alternatively be the voltage level across the sensor, or across a different LED in the sensor, for example. In some cases, the sensor 14 may contain multiple emitters and/ or multiple LEDs. The voltage measured may be the voltage drop across any one or more of the LEDs and/or emitters in the sensor, for This same measurement can occur at multiple current levels. These levels could be, for example 20 or 25 mA. In some cases, the utilized currents could be higher or lower, such as 10 or 15 mA or 40 or 45 or 50 mA. As seen in decision 88, if the actual current level and the measured forward voltage are both within a tolerance, then the algorithm may proceed. If the actual measured current varies from the first current by more than a tolerance, or the measured forward voltage varies from a target by more than a tolerance, then the patient monitor 12 may issue an alarm 90. This could be an audible or visual alarm, an instruction to the algorithm for further processing, or a change in operating, for example.

In step 92 the patient monitor 12 will use the measured voltage and measured current data to determine a maximum drive current value for the patient monitor 12 in combination with the sensor 14. This could be an absolute maximum value, or an artificial maximum value that includes some buffer or headroom to ensure that the LED can be run safely and effectively. The measured values may include both current and voltage measured at two current levels, such as 20 and 25 mA. These current and voltage measurements are used to create a V/I curve for the sensor, which is compared to a projection of the supply line voltage for the patient monitor, for example, to determine the maximum current value available to the sensor.

In step 94, the measured current and voltage values are used to set parameters for the patient monitor 12 to carry out monitoring of a patient. For example, in pulse oximetry, it may be necessary to vary the current to one or more LEDs in the sensor 14, such as the red LED in order to improve measurement capabilities of the patient monitor. The measured current and/or voltage values can help inform the patient monitor 12 of specific characteristics of the sensor 14 and/or LEDs, allowing the patient monitor 12 to more effectively vary the output of the sensor 14 and/or LEDs. This could be done by defining the slope and offset of the V/I curve of one or more LEDs, for example.

In step 96, the patient monitor 12 drives the LED to the projected maximum current. If the actual current level does not reach the maximum current level, or some tolerance from the maximum current level, then the patient monitor 12 can signal an alarm 100 at decision 98. This could be audible or visual, or some other alarm. It could be the same or similar alarm as alarm 90, or different. Alternatively, the alarm could be a diversion to optional step 110, in which the target current may be adjusted and then the algorithm can re-test the maximum current.

In step 102, the voltage is measured with the current being driven to the maximum current level. In decision 104, if the voltage is not within tolerance, the patient monitor 12 could alarm 106. As in decision 98, this could be any type of alarm, including a diversion to step 110.

An additional decision 108 could be made, to verify that buffer or headroom is correct. The buffer or headroom is an additional voltage or current adjustment beyond the actual measurements, to avoid operational space that is inconsistent within the LED. For example, if a maximum current for a particular combination of patient monitoring components 120 and sensor 14 is determined to be 25mA, the buffer value could be set to 3mA, which would then result in a maximum current value of 22mA. The area between 22mA and 25mA might still be operational in the sense that the LED will function, but the results might be inconsistent. The buffer or headroom could be set as a fixed value, or could vary, as discussed above. If the headroom is insufficient, and/or current measured in step 96 is determined not to be correct, then the maximum current is lowered in step 110, and the process of testing the maximum current can be repeated. When a maximum current is found that satisfies the headroom requirement as well as the current and voltages requirements, the patient monitoring system 10 can proceed with monitoring the patient.

In an embodiment, the patient monitor 12 reduces the operating range of the LED drive 60 to match the operating range of the sensor 14, based on the information encoded on the sensor. For example, a patient monitor may be programmed to operate older-generation sensors between 0 and 50 mA, without any constraints on that range. This could be sufficient for older sensors with LED's that operate at lower voltages, such as LED A in Figure 6. However, when a newer sensor with an encoded check level is connected to the monitor, the monitor reduces the operating range of the LED drive 60 based on that encoded information. Specifically, the LED drive may be adjusted to raise the minimum to an encoded minimum value for that sensor, and to reduce the maximum to either the check level encoded on the sensor or to a projected maximum level based on the maximum voltage available from the monitor, as described above. For example, the LED drive may be adjusted to drive the sensor between 4 mA (the minimum to operate the LED's) and 25 mA (the check level encoded on the sensor, or the projected maximum current based on the available voltage). In an embodiment, the sensor 14 is a wireless sensor with its own on-board battery, and the check value encoded on the sensor is used to adjust the LED drive 60 to operate the sensor within a current range that can be delivered by the battery. In an embodiment, when a sensor is connected to a monitor, the monitor reads the values encoded on the sensor, including a check current level, and then attempts to drive the sensor to that check current level. If successful, the sensor is compatible and can operate within a range between its minimum and the check value. If the monitor cannot reach that check value, perhaps due to voltage limits, then the sensor is rejected.

In an embodiment, the sensor 14 may include an encoder 42 as shown in Fig. 2. This encoder may store data that could help with the functioning of the patient monitor 12 operation as described above. By way of example, the encoder 42 could store data related to one or more LEDs, such as the wavelength of the LEDs, or calibration coefficients for the LEDs. These calibration coefficients could adjust a monitoring algorithm to be more accurate for a particular set of one or more LEDs. The calibration coefficients could be a single set of coefficients for all operations of the patient monitor, or the encoder 42 could store more than one set of calibration coefficients to calibrate the patient monitor 12 for use with the LEDs in multiple scenarios. By way of example, in a pulse oximetry monitor, the encoder 42 may include one set of calibration coefficients to calibrate the patient monitor 12 when the oxygenation of a patient is in a normal range, for example above 80%. The encoder 42 may also include data indicating where the change may happen, for instance, in this case 80%. The encoder 42 may also include a second set of calibration coefficients to calibrate the patient monitor 12 when the oxygenation of a patient is below 80%, for example.

Additionally, encoder 42 may include data related to the relationship between emitter drive line capacities and the LED input requirements as discussed above. This data may include a minimum drive current that can be used by the emitter drive line to drive an LED. LEDs may have different input requirement characteristics, so this minimum current could change between individual LEDs, or models of LED for example. The data in the encoder 42 could, by way of example, include a maximum current at which an LED should be driven by the emitter drive line. Again, this could be individual to the particular LED or to the line of sensors, or some other grouping. Alternatively, or additionally, the encoder 42 could include data on the minimum allowable maximum drive current. This data could, for example, be a numerical indication of a current that the patient monitor 12 could use as a threshold for alarming, or for another purpose. The encoder 42 could further include data on the correlation between the forward voltage of an emitter and temperature.

According to an embodiment, the encoder 42 could include additional or different information helpful to the patient monitor 12 to ensure that the patient monitor 12 can safely and effectively drive one or more LEDs in the sensor. For example, an index could be created, that incorporates one or more input parameters for the sensor 14 along with one or more requirements for drive power from the emitter drive line. This could be stored in the form of a number that the patient monitor 12 could then utilize to determine whether the emitter 16 is compatible with the patient monitor 12 as it is implemented.

The encoder 42 could include the voltage required to drive one or more LEDs at one or more drive currents. This voltage could be measured during manufacture of the sensor, for example.

In an embodiment, the voltage required to drive one LED at two different drive currents could be measured at the time of manufacture, and recorded on the encoder 42. During use, the patient monitor 12 could also test the LED input requirements. The patient monitor 12 could then compare the voltages included in encoder 42 with the voltages determined at the time of use in order to verify that the sensor values have not changed. If the sensor values have changed, the patient monitor 12 could alarm by providing an auditory or visual alarm, terminating operations, or some other alarm.

In this way, the patient monitor 12 could protect against several sources of sensor error. For example, sometimes an encoder could be physically taken from an existing sensor 14 and reused in a new sensor 14 with different optical components. Alternatively, the contents of the encoder 42 could be copied into another encoder 42 in a new sensor 14 with new optical components. As discussed herein, the safety and efficacy of the patient monitoring system 10 can be compromised by bypassing these safety features. By verifying the values in the encoder 42, this error may be avoided. Additionally, the optical components may become damaged during manufacture, transport and/or storage. Verifying the encoder 42 matches the existing optical components within the sensor 14 may help to avoid sensor error.

Additionally, the forward voltage across the emitter can continue to be measured during the patient monitoring process. To ensure patient safety, the safety check procedure ensures that a monitor and sensor can operate together with a wide enough operating range that the combination can effectively (and safely) monitor a patient. When dealing with emitters in contact with, or near to patient tissue, the upper bounds of safety can be dictated by the heat produced by the emitter. According to an embodiment, the RED LED in a pulse oximetry sensor can produce heat that could burn a patient if driven with too much power. In order to maximize the power that can be used to drive the emitter (and thus maximize the operating range of the system), the temperature of the emitter can be monitored. This can occur by placing a temperature sensor in proximity of the emitter to constantly measure the temperature of the emitter. This temperature can then be used to control the maximum power (or current or voltage) supplied to the emitter drive line. Alternatively, the forward voltage across the emitter correlates closely to the actual temperature of the emitter. According to an embodiment, the forward voltage can be measured during operation of the monitor and sensor combination to track the temperature of the emitter, and this temperature result can be used to control the maximum power to be supplied to the emitter. To facilitate this, the correlation between forward voltage and temperature can be stored in the sensor with the sensor data and utilized by the monitor during operation. According to an embodiment, this temperature data from the temperature sensor or from the forward voltage measurement can be used to identify periods when the sensor has become detached from the patient, and an alarm can be activated.

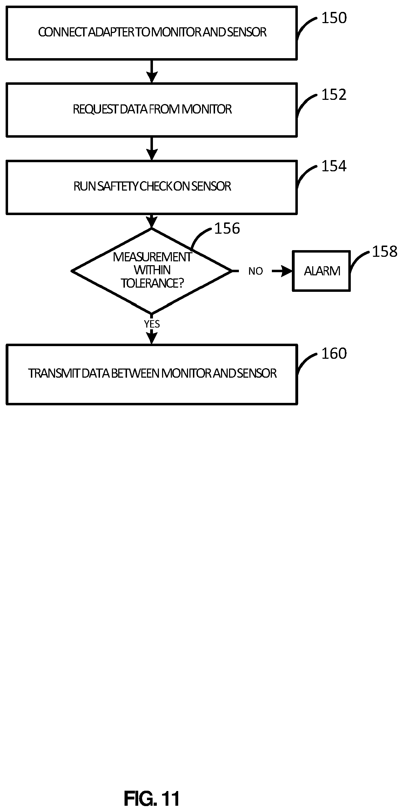

As discussed above, both sensors and monitors advance as technology advances. According to embodiments, the developments described above help to ensure that sensor and monitor are adequately compatible, so that the combination will function correctly. In some cases, a sensor or monitor will be used that was not designed with the features discussed herein, i.e. the sensor or monitor will not be designed with a safety check process as described above. According to an embodiment, a system such as that shown in Figure 11 can allow the system to undergo a safety check process even when part of the system was not designed for it.

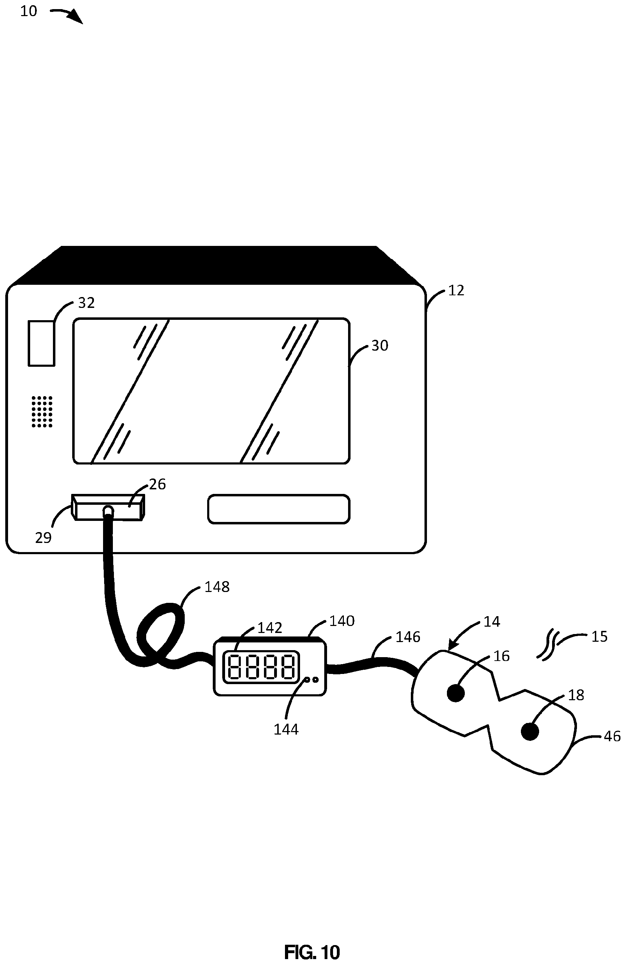

In Figure 10, a monitor 12 is shown, similar to the monitor 12 shown in Figure 1. In between the monitor 12 and the sensor 14, and communicatively coupled to each, is an adapter 140. In the embodiment shown, wire 146 and wire 148 serve to communicatively couple the adapter 140 with the sensor 14 and the monitor 12, respectively. It should be understood that one or both of the wires 146 and 148 could be replaced with a wireless communication link, or some other communication link. Wires 146 and/or 148 may be detachable from the adapter 140, the sensor 14 and/or the monitor 12. Similarly, the wires 146 or 148 may be omitted if the functionality of the adapter is incorporated into the sensor 14 or the monitor 12, respectively.

Adapter 140 includes an LCD display 142, and LED indicators 144. It should be understood that these indicators are optional, and could be replaced by any other type of display, such as CRT, LED, ELD, E Ink, PDP, OLED, DLP, SED, FED, laser TV, carbon nanotubes, quantum dot, IMOD, or DMS, or could be eliminated entirely. Adapter 140 could further include various buttons or switched as desired.

Figure 11 shows a method of operating a system including a monitor 12 that is not designed to complete a safety check as described above. In Step 150, the adapter 140 is connected to a sensor 14, and the adapter 140 is connected to a monitor 12. The adapter 140 may be designed to prefer or require that either the sensor 14 or monitor 12 be connected first. The adapter could signal an alarm if the incorrect sequence is used. This alarm could be an indication on the LCD display 142 or an indicator 144, or some other alarm including that the adapter 140 could simply not function, or function only partially until the correct procedure is followed. In Step 152, the adapter 140 then requests data from the monitor 12. This data may include model information, sensor requirements, emitter drive characteristics, parameter information, historical information or the like. According to an embodiment, the monitor 12 may send this information to the adapter 140 without waiting for a request. This data may be information that the monitor 12 is designed to send to a sensor 14 or adapter 140, or it may be data that the adapter 140 gathers from the monitor's operation or in some other way. According to an embodiment, the data allows the adapter 140 to characterize the drive line of the monitor 12 and/or enable communication between a monitor 12 and sensor 14, whether or not they were designed to communicate with each other.

In response to the data received from the monitor 12, the adapter 140 can determine an appropriate check level for the sensor 14. The adapter then runs the safety check as described above in Step 154. In response to the safety check, in Step 156, the adapter 140 determines whether the sensor 14 is within tolerance to operate with the monitor 12. If the sensor 14 is not compatible with the monitor12, the adapter 140 will alarm. As discussed above, this alarm could take many forms, such as an audible, visual, or aural alarm. Additionally, it may take the form of not allowing the monitor and sensor to operate together, either in whole or in part.

In Step 160, if the sensor 14 does pass the safety check, the adapter 140 can function as an intermediary between the monitor 12 and the sensor 14. This function as intermediary could mean translating data - drive line signals and sensor signals, for example - so that the sensor 14 and monitor 12 can communicate. The function as intermediary may include a handshake communication with the monitor 12 and/or the sensor 14 to identify the monitor 12 or sensor 14 to the other. The function may include inserting information to falsely identify the monitor 12 or sensor 14 to the other, such that the monitor 12 and sensor 14 will communicate with each other even if they were designed not to communicate.

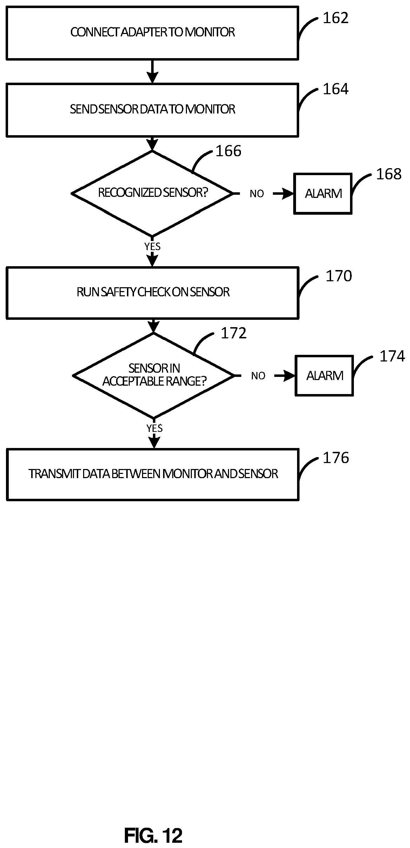

Turning to Figure 12, a method is shown in which a monitor that is designed to operate a safety check works with a sensor that may not be designed for a safety check. In Step 162, the adapter 140 is connected to the monitor 12. As above, the order of connecting the monitor 12 and sensor 14 to the adapter is not important, although an implementation may require a particular order. According to an embodiment, when the sensor 14 is connected to the adapter 140, the adapter 140 receives information from the sensor 14 which allows the adapter 140 to characterize the sensor 14. This information may include sensor identification data, sensor type data, emitter information, detector information, manufacturing information, connector information, or any other information that enables the adapter 140 to connect the sensor 14 with the monitor 12 and/or allows the monitor 12 to run a safety check on the sensor 14. In Step 164, the adapter 140 sends data to the monitor 12 in a format that the monitor 12 expects or requests. According to an embodiment, the adapter 140 will send data to the monitor 12 such that the adapter 140 will appear invisible to the monitor 12, and the monitor 12 will perceive that is communicating with a sensor 14 with which the monitor 12 was designed to operate.

In Step 166, the monitor 12 will determine whether the sensor 14 appears to be a recognized sensor. In an embodiment utilizing adapter 140, this determination can based on whether the adapter 140 is able to communicate with the sensor 14 in order to provide characterization that will allow monitor 12 to properly test the compatibility of the sensor through the safety check described above. This could include sensors that were designed to pass through the safety check, including those with V/I information stored on the sensor 14. Sensors that were not designed to pass through a safety check and/or do not have V/I information stored on the sensor 14 could also be included is the adapter 140 is able to characterize the sensor 14, such as through a sensor type, emitter type, etc. In this way, the adapter can facilitate communications between sensors and monitors that were not designed to work together, while still allowing for a safety check to ensure that the combination of monitor 12 and sensor 14 will be safe and effective for the patient and caregiver.

In Step 168, the monitor 12 or adapter 140 produce an alarm if the sensor 14 is not recognized by the monitor 12 and the adapter 140 is not able to characterize the sensor 14 sufficiently. The alarm could be visual, audible or tactile, or the alarm could be a lack of or limitation of functionality.

In Step 170, the monitor 12 runs the safety check as described above. If the sensor 14, as adjusted through the adapter 140, is in an acceptable range, then the monitor 12 and sensor 14 will proceed to function together with the adapter 140 translating and/or passing information between the two in Step 176. If the sensor 14 is not in an acceptable range, then in Step 174 the monitor 12 or adapter 140 will produce an alarm as described above.

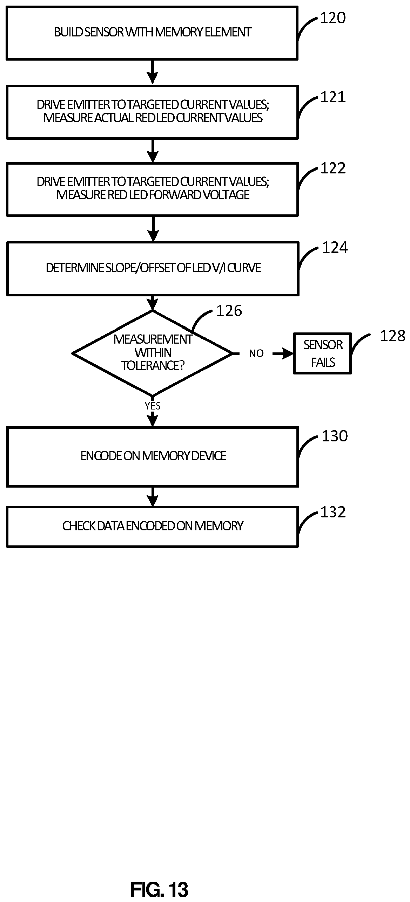

Figure 13 shows a method of manufacturing a sensor according to an embodiment. In Step 120 a sensor is built with a memory element, such as encoder 42. The sensor has at least one emitter, such as a red LED. According to an embodiment, the sensor could be a pulse oximetry sensor with red and infrared LEDs. According to various embodiments, the sensor could take any number of forms. The sensor could have, for example, a bandage-type wrap, a structural case, multiple LEDs with various wavelengths, electrodes, a temperature sensor, a motion sensor, wired communications, wireless communications, on board processing, etc.

In Step 121, the emitter is driven with a target drive current. The actual drive current may vary slightly based on the individual physical properties of the emitter. While the emitter is driven with a target drive current, the actual drive current can be measured. In Step 122, while the emitter is being driven at the target drive current, the actual forward voltage of the emitter is measured. Steps 121 and 122 can be repeated for multiple target drive currents. In this way, the V/I curve of the emitter can be determined. In an embodiment, one or more target drive currents can be used together with known manufacturing hardware to determine how the emitter varies from known emitter specifications, enabling determination of the V/I curve for the emitter.

In Step 124, The V/I curve for the emitter can be determined as a complete curve, as one or more points on a curve, as one or more points together with one or more slopes of a line or curve, or in some other way. For example, the V/I curve can be represented by a slope and offset of a line.

In Step 126, the V/I curve determined in Step 124 is compared to a reference value to determine whether the sensor is within an acceptable tolerance. This tolerance can be preset based on sensor and/or emitter specifications, for example. If the V/I curve is not within an acceptable tolerance, the sensor is rejected in Step 128. The sensor could then be sent back for repair/reprocessing, or could be labeled for restricted use, or rejected in some other way. If the V/I curve is within an acceptable tolerance, then in Step 130 the V/I curve is recorded onto the memory, such as encoder 42. As above, this V/I curve can be recorded in a variety of formats, such as one or a plurality of points, a slope and/or offset, or in some other way. In Step 132, the data that was recorded onto the memory is verified for accuracy.

The systems and methods described here may be provided in the form of tangible and non-transitory machine-readable medium or media (such as a hard disk drive, hardware memory, etc.) having instructions recorded thereon for execution by a processor or computer. The set of instructions may include various commands that instruct the computer or processor to perform specific operations such as the methods and processes of the various embodiments described here. The set of instructions may be in the form of a software program or application. The computer storage media may include volatile and non-volatile media, and removable and non-removable media, for storage of information such as computer-readable instructions, data structures, program modules or other data. The computer storage media may include, but are not limited to, RAM, ROM, EPROM, EEPROM, flash memory or other solid state memory technology, CD-ROM, DVD, or other optical storage, magnetic disk storage, or any other hardware medium which may be used to store desired information and that may be accessed by components of the system. Components of the system may communicate with each other via wired or wireless communication. The components may be separate from each other, or various combinations of components may be integrated together into a medical monitor or processor, or contained within a workstation with standard computer hardware (for example, processors, circuitry, logic circuits, memory, and the like). The system may include processing devices such as microprocessors, microcontrollers, integrated circuits, control units, storage media, and other hardware.

Although the present invention has been described and illustrated in respect to exemplary embodiments, it is to be understood that it is not to be so limited, since changes and modifications may be made therein which are within the full intended scope of this invention as hereinafter claimed.

## Claims

1. A method of initializing a sensor (14) for monitoring a patient, comprising:
driving, by a patient monitor (12), an oximetry sensor (14) at a first current level, the oximetry sensor (14) comprising an LED (16);
measuring, by the patient monitor (12), a first voltage across the LED (16) at the first current level;
driving, by the patient monitor (12), the pulse oximetry sensor (14) at a second current level;
measuring, by the patient monitor (12), a second voltage across the LED (16) at the second current level; and
determining, by the patient monitor (12), a voltage/current curve for the LED (16) using the measured first voltage and measured second voltage;
**characterised in that** the method further comprises:
projecting, by the patient monitor (12) a next sensor voltage based at least on the first and second voltages by projecting the voltage/current curve for the light emitter to a point that it intersects a voltage/current curve for voltage available from the patient monitor (12) to drive the LED (16);
identifying a third current level that could produce the projected next sensor voltage, wherein the third current level is a maximum emitter drive current that can be provided by the patient monitor (12) to the LED (16) in the sensor (14);comparing, by the patient monitor (12), the determined maximum emitter drive current to a check value for the sensor (14), the check value comprising a lowest acceptable maximum current for driving the LED (16); and
producing an alarm in response to the comparison.

2. The method of claim 1 wherein the next sensor voltage is a voltage that is above a voltage threshold.

3. The method of claim 1 wherein the step of projecting includes analyzing a circuit model for the sensor (14).

4. The method of claim 1 wherein the sensor (14) includes a red LED and an infrared LED.

5. The method of claim 4 wherein the sensor voltage is a voltage across the red LED with the current passing through the red LED.

6. The method of claim 4 wherein the sensor voltage is a voltage across the infrared LED with the current passing through the infrared LED.

7. The method of claim 1 wherein the alarm is an indication that the sensor (14) is incompatible with a patient monitor (12).

8. The method of claim 1 wherein the alarm is an indication of a maximum drive current that can be provided to the sensor (14) by a patient monitor (12).

9. The method of claim 1 wherein the step of projecting includes analyzing capabilities of a patient monitor (12).

10. A patient monitoring system, comprising:
a sensor (14), comprising:
an LED (16);
a patient monitor (12), comprising:
circuitry configured to measure a plurality of voltage levels across the LED (16) at a plurality of different drive current levels; and
a processor configured to determine an emitter drive current threshold based on at least one of the plurality of sensor voltage levels;
**characterised in that** the emitter drive current threshold is a maximum emitter drive current that can be provided by the patient monitor (12) to the LED (16) in the sensor (14).

11. The patient monitoring system of claim 10, wherein the patient monitor (12) further comprises a supply line voltage, and the processor utilizes the supply line voltage to determine the emitter drive current threshold.

12. The patient monitoring system of claim 10, wherein the processor further produces an alarm based on the emitter drive current threshold.

13. The patient monitoring system of claim 12, wherein the alarm limits functionality of the patient monitor (12) and/or the alarm limits functionality of the sensor (14).

## Patentansprüche

1. Verfahren zum Initialisieren eines Sensors (14) zum Überwachen eines Patienten, umfassend:
Antreiben, mittels eines Patientenmonitors (12), eines Oximetriesensors (14) auf einem ersten Stromniveau, der Oximetriesensor (14) umfassend eine LED (16);
Messen, mittels des Patientenmonitors (12), einer ersten Spannung über der LED (16) auf dem ersten Stromniveau;
Antreiben, mittels des Patientenmonitors (12), des Pulsoximetriesensors (14) auf einem zweiten Stromniveau;
Messen, mittels des Patientenmonitors (12), einer zweiten Spannung über der LED (16) auf dem zweiten Stromniveau; und
Bestimmen, mittels des Patientenmonitors (12), einer Spannungs-/Stromkurve für die LED (16) unter Verwendung der gemessenen ersten Spannung und der gemessenen zweiten Spannung;
**dadurch gekennzeichnet, dass** das Verfahren ferner umfasst:
Projizieren, mittels des Patientenmonitors (12), einer nächsten Sensorspannung basierend mindestens auf der ersten und der zweiten Spannung mittels Projizieren der Spannungs-/Stromkurve für den Lichtemitter zu einem Punkt, dass sie eine Spannungs-/Stromkurve für die Spannung schneidet, die von dem Patientenmonitor (12) verfügbar ist, um die LED (16) anzutreiben;
Identifizieren eines dritten Stromniveaus, der die projizierte nächste Sensorspannung erzeugen könnte, wobei das dritte Stromniveau ein maximaler Emitterantriebsstrom ist, der von dem Patientenmonitor (12) an die LED (16) in dem Sensor (14) bereitgestellt werden kann; Vergleichen, mittels des Patientenmonitors (12), des bestimmten maximalen Emitterantriebsstroms mit einem Prüfwert für den Sensor (14), der Prüfwert umfassend einen niedrigsten akzeptablen maximalen Strom zum Antreiben der LED (16); und
Erzeugen eines Alarms als Reaktion auf den Vergleich.

2. Verfahren nach Anspruch 1, wobei die nächste Sensorspannung eine Spannung ist, die über einem Spannungsschwellenwert liegt.

3. Verfahren nach Anspruch 1, wobei der Schritt des Projizierens ein Analysieren eines Schaltungsmodells für den Sensor (14) einschließt.

4. Verfahren nach Anspruch 1, wobei der Sensor (14) eine rote LED und eine Infrarot-LED einschließt.

5. Verfahren nach Anspruch 4, wobei die Sensorspannung eine Spannung über der roten LED ist, wobei der Strom durch die rote LED fließt.

6. Verfahren nach Anspruch 4, wobei die Sensorspannung eine Spannung über der Infrarot-LED ist, wobei der Strom durch die Infrarot-LED fließt.

7. Verfahren nach Anspruch 1, wobei der Alarm eine Angabe ist, dass der Sensor (14) mit einem Patientenmonitor (12) inkompatibel ist.

8. Verfahren nach Anspruch 1, wobei der Alarm eine Angabe eines maximalen Antriebsstroms ist, der dem Sensor (14) mittels eines Patientenmonitors (12) bereitgestellt werden kann.

9. Verfahren nach Anspruch 1, wobei der Schritt des Projizierens das Analysieren von Fähigkeiten eines Patientenmonitors (12) einschließt.

10. Patientenüberwachungssystem, umfassend:
einen Sensor (14), umfassend:
eine LED (16);
einen Patientenmonitor (12), umfassend:
Schaltungen, die konfiguriert sind, um eine Vielzahl von Spannungsniveaus über die LED (16) bei einer Vielzahl von unterschiedlichen Antriebsstromniveaus zu messen; und
einen Prozessor, der konfiguriert ist, um einen Emitterantriebsstromschwellenwert basierend auf mindestens einem der Vielzahl von Sensorspannungsniveaus zu bestimmen;
**dadurch gekennzeichnet, dass** der Emitterantriebsstromschwellenwert ein maximaler Emitterantriebsstrom ist, der mittels des Patientenmonitors (12) an die LED (16) in dem Sensor (14) bereitgestellt werden kann.

11. Patientenüberwachungssystem nach Anspruch 10, wobei der Patientenmonitor (12) ferner eine Versorgungsleitungsspannung umfasst und der Prozessor die Versorgungsleitungsspannung verwendet, um den Emitterantriebsstromschwellenwert zu bestimmen.

12. Patientenüberwachungssystem nach Anspruch 10, wobei der Prozessor ferner einen Alarm basierend auf dem Emitterantriebsstromschwellenwert erzeugt.

13. Patientenüberwachungssystem nach Anspruch 12, wobei der Alarm die Funktionalität des Patientenmonitors (12) begrenzt und/oder der Alarm die Funktionalität des Sensors (14) begrenzt.

## Revendications

1. Procédé d'initialisation d'un capteur (14) destiné à surveiller un patient, comprenant :
l'entraînement, par un dispositif de surveillance de patient (12), d'un capteur d'oxymétrie (14) à un premier niveau de courant, le capteur d'oxymétrie (14) comprenant une DEL (16) ;
la mesure, par le dispositif de surveillance de patient (12), d'une première tension aux bornes de la DEL (16) au premier niveau de courant ;
l'entraînement, par le dispositif de surveillance de patient (12), du capteur d'oxymétrie de pouls (14) à un deuxième niveau de courant ;
la mesure, par le dispositif de surveillance de patient (12), d'une seconde tension aux bornes de la DEL (16) au deuxième niveau de courant ; et
la détermination, par le dispositif de surveillance de patient (12), d'une courbe tension/courant pour la DEL (16) à l'aide de la première tension mesurée et la seconde tension mesurée ;
**caractérisé en ce que** le procédé comprend en outre :
la projection, par le dispositif de surveillance de patient (12), d'une tension de capteur suivante sur la base au moins des première et seconde tensions en projetant la courbe tension/courant pour l'émetteur de lumière jusqu'à un point où elle coupe une courbe tension/courant pour la tension disponible depuis le dispositif de surveillance de patient (12) pour entraîner la DEL (16) ;
l'identification d'un troisième niveau de courant qui pourrait produire la tension de capteur suivante projetée, le troisième niveau de courant étant un courant d'entraînement maximal d'émetteur qui peut être fourni par le dispositif de surveillance de patient (12) à la DEL (16) dans le capteur (14) ;la comparaison, par le dispositif de surveillance de patient (12), du courant d'entraînement maximal d'émetteur déterminé à une valeur de contrôle pour le capteur (14), la valeur de contrôle comprenant un courant maximal acceptable le plus faible pour entraîner la DEL (16) ; et
le déclenchement d'une alarme en réponse à la comparaison.

2. Procédé selon la revendication 1, dans lequel la tension de capteur suivante est une tension qui est supérieure à un seuil de tension.

3. Procédé selon la revendication 1, dans lequel l'étape de projection comporte l'analyse d'un modèle de circuit pour le capteur (14).

4. Procédé selon la revendication 1, dans lequel le capteur (14) comporte une DEL rouge et une DEL infrarouge.

5. Procédé selon la revendication 4, dans lequel la tension de capteur est une tension aux bornes de la DEL rouge avec le courant traversant la DEL rouge.

6. Procédé selon la revendication 4, dans lequel la tension de capteur est une tension aux bornes de la DEL infrarouge avec le courant traversant la DEL infrarouge.

7. Procédé selon la revendication 1, dans lequel l'alarme est une indication que le capteur (14) est incompatible avec un dispositif de surveillance de patient (12).

8. Procédé selon la revendication 1, dans lequel l'alarme est une indication d'un courant d'entraînement maximal qui peut être fourni au capteur (14) par un dispositif de surveillance de patient (12).

9. Procédé selon la revendication 1, dans lequel l'étape de projection comporte l'analyse de capacités d'un dispositif de surveillance de patient (12).

10. Système de surveillance de patient, comprenant :
un capteur (14) comprenant :
une DEL (16) ;
un dispositif de surveillance de patient (12), comprenant :
des circuits configurés pour mesurer une pluralité de niveaux de tension aux bornes de la DEL (16) à une pluralité de niveaux de courant d'entraînement différents ; et
un processeur configuré pour déterminer un seuil de courant d'entraînement d'émetteur sur la base d'au moins l'un de la pluralité de niveaux de tension de capteur ;
**caractérisé en ce que** le seuil de courant d'entraînement d'émetteur est un courant d'entraînement d'émetteur maximal qui peut être fourni par le dispositif de surveillance de patient (12) à la DEL (16) dans le capteur (14).

11. Système de surveillance de patient selon la revendication 10, dans lequel le dispositif de surveillance de patient (12) comprend en outre une tension de ligne d'alimentation, et le processeur utilise la tension de ligne d'alimentation pour déterminer le seuil de courant d'entraînement d'émetteur.

12. Système de surveillance de patient selon la revendication 10, dans lequel le processeur déclenche en outre une alarme sur la base du seuil de courant d'entraînement d'émetteur.

13. Système de surveillance de patient selon la revendication 12, dans lequel l'alarme limite la fonctionnalité du dispositif de surveillance de patient (12) et/ou l'alarme limite la fonctionnalité du capteur (14).
